# EUROPEAN PATENT SPECIFICATION

(11) **EP 2 736 580 B1**
(45) Date of publication and mention of the grant of the patent: **18.09.2019**
(21) Application number: 12743337.3
(22) Date of filing: 26.07.2012
(51) Int. Cl.: A61M 25/00, A61M 25/06, A61F 2/04, A61M 25/09, A61M 27/00

(54) **RAPID EXCHANGE STENT DELIVERY SYSTEM**
TRÄGERSYSTEM FÜR RX-STENT
SYSTÈME DE POSE D'ENDOPROTHÈSE À ÉCHANGE RAPIDE

(30) Priority: 29.07.2011 US 201161513101 P
(43) Date of publication of application: 04.06.2014
(73) Proprietor: Boston Scientific Scimed, Inc., Maple Grove, MN 55311 (US)
(72) Inventor: FERNANDES, Alex, J., Marlboro, MA 01752 (US); LEANNA, Gary, J., Holden, Massachusetts 01520 (US); AMOS, Michael, Devon, Boston, Massachusetts 02118 (US); HOLLETT, Andrew, K., Waltham, Massachusetts 02453 (US)
(74) Representative: Vossius & Partner Patentanwälte Rechtsanwälte mbB
(86) International application number: PCT/US2012/048323
(87) International publication number: WO 2013/019550

(56) References cited:
- EP-A2- 0 792 657
- WO-A1-99/59664
- US-B1- 6 264 624

## Description

### FIELD OF THE INVENTION

The present invention pertains to medical devices. More particularly, the present invention pertains to medical devices for delivering stents to the biliary tract and/or the pancreatic tract.

### BACKGROUND

A wide variety of intraluminal medical devices have been developed for medical use, for example, use in the biliary tract. Some of these devices include guidewires, catheters, stents, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

WO0069498 discloses a drainage catheter delivery system for use with a conventional guidewire and includes a push catheter, a stent and a retention device for selectively secure the stent to the push catheter.

EP2067454 discloses a stent delivery system which is inserted into an endoscope and is for placing a stent inside a body cavity; the catheter includes a stent housing for housing at least a part of the stent provided in a unitary manner to the front end of the pusher catheter 11.

WO 99/59664 A1 discloses a locking device that is mounted on an endoscope or the like for selectively securing the position of a guide wire and/or catheter relative to the endoscope or the like. The locking device preferably includes a side wall with an opening therein for receiving the proximal end of a guide wire or catheter. The opening is preferably J-shaped or boot shaped, and has an entry end and a locking end. Once a guide wire or catheter is in a desired position within a body cavity, the portion of the guide wire or catheter that extends outside of the endoscope or the like may be moved into the opening.

### BRIEF SUMMARY

According to the invention, an assembly as recited in the independent claim is provided. The dependent claims define embodiments. Any eventual references to an "embodiment" or "embodiments" throughout the description which are not under the scope of the defined claims merely represent possible examples and are therefore not part of the present invention.

The present disclosure provides design, material, manufacturing method, and use alternatives for medical devices or components thereof. An assembly according to an embodiment comprises a combination device that may function as an anchoring mechanism cover and a guidewire lock. The combination device includes a tubular body. The tubular body defines an inner diameter and may have a locking portion. The inner diameter is sized to fit about an outer diameter of a drainage stent having an anchoring mechanism. The locking portion is configured to secure the position of a guidewire relative to a catheter system.

The assembly may be a stent delivery system and includes a guidewire. A guide catheter may be disposed about the guidewire. A push catheter may be disposed over the guide catheter. A drainage stent may be disposed over the guide catheter. The stent has an anchoring mechanism. The system also includes a combination device for covering the anchoring mechanism of the stent and for securing the position of the guidewire relative to the push catheter. The combination device includes a tubular body having a locking portion formed therein.

Also disclosed is an example medical device assembly that may include a first medical device. A second medical device may be disposed within at least a portion of the first medical device. A guidewire may extend through at least a section of the first medical device, the second medical device, or both. The assembly may also include a combination device for loading the second medical device into the first medical device and for securing the position the guidewire relative to the second medical device. The combination device may be disposed about the second medical device and may be movable along the second medical device.

The above summary of some embodiments is not intended to describe each disclosed embodiment or every implementation of the present invention. The Figures and Detailed Description which follow more particularly exemplify these embodiments.

### BRIEF DESCRIPTION OF THE DRAWINGS

The invention may be more completely understood in consideration of the following detailed description of various embodiments of the invention in connection with the accompanying drawings, in which:
Figure 1 is a plan view of an example catheter system;
Figure 2 is partial cross-sectional side view of the catheter system shown in Figure 1;
Figure 3 is a side view of an example combination device;
Figure 4 is a side view of another example combination device;
Figure 5 is a side view of another example combination device;
Figure 6 illustrates the use of an example combination device to compress a flap formed on a drainage stent so that the stent may be loaded into a medical device;
Figures 7-9 illustrate the use of an example combination device to secure the position of a guidewire relative to a catheter system; and
Figure 10 is a side view of another example combination device.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the invention as defined by the claims.

### DETAILED DESCRIPTION

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The drawings, which are not necessarily to scale, depict illustrative embodiments and are not intended to limit the scope of the invention.

A wide variety of biliary, endoscopic, and/or endosurgical procedures have been developed for making medical treatments, diagnoses, and images of areas along the biliary tract and/or the pancreatic tract. For the purposes of this disclosure, the "biliary tract" and/or the "pancreatic tract" are understood to include various components of the digestive system and include, for example, the various ducts of the biliary tree between the liver and the duodenum as well as the various ducts between the pancreas and the duodenum. Numerous endoscopic and/or endosurgical devices have been developed for making medical treatments, diagnoses, and images of areas along the biliary and pancreatic tracts. Some of these device and/or procedures include biliary catheters, biliary guidewires, biliary stent delivery systems, and the like. In general, these devices are guided to the biliary and/or pancreatic tract by an endoscope (and/or a duodenoscope, sheath, guide tube, catheter, etc.) that is disposed in the duodenum. Once positioned, various interventions can be performed depending on the needs of the patient and the type of device utilized. Other locations and/or uses are also contemplated for the systems disclosed herein including, for example, urinary tract interventions and/or urological interventions, gynecological interventions, etc.

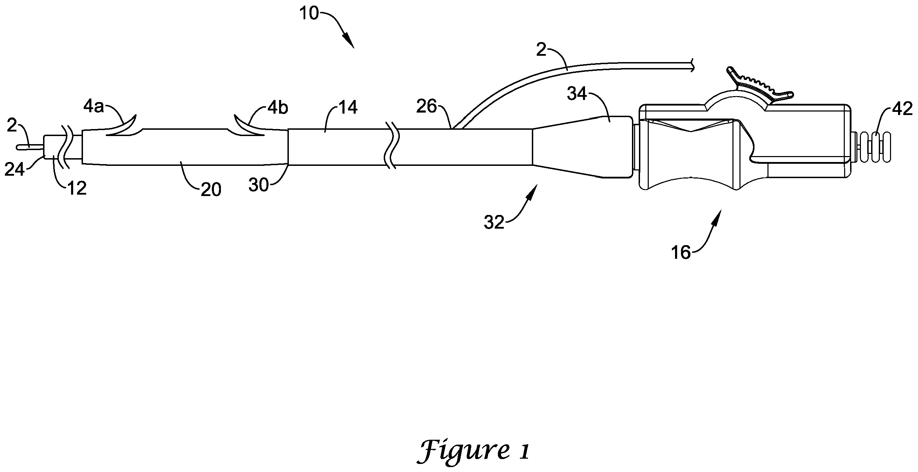

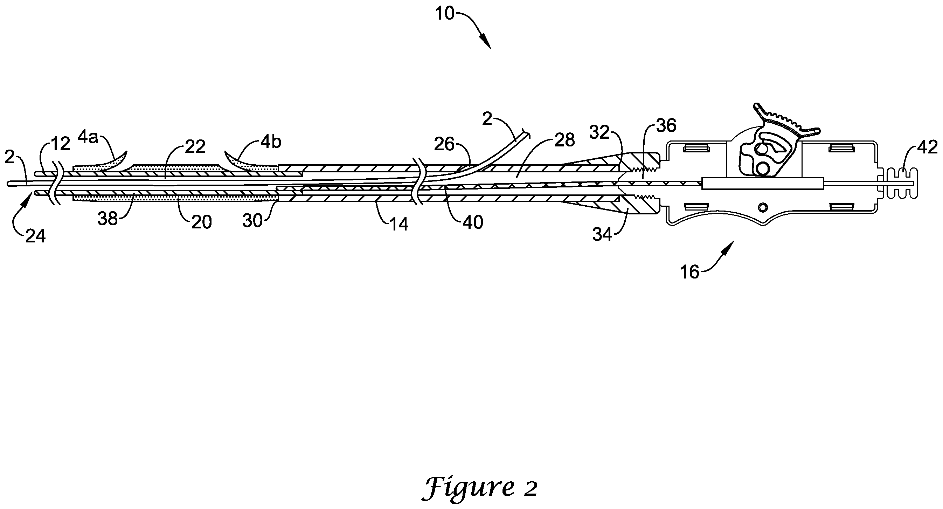

Referring now to Figures 1 and 2, there is shown an example medical device, illustrated as a catheter and/or delivery system 10 that may be used, for example, for delivering a stent 20 such as a drainage stent to a suitable target location such as, for example, a target along the biliary and/or pancreatic tree. The system 10 may also be used at any other suitable location. The stent 20 may be used to bypass or drain an obstructed lumen, for example along the biliary and/or pancreatic tree, and can be configured for long-term positioning within the body. It should be understood that the terms "drainage stent", "drainage catheter" and "stent" can be used interchangeably with reference to the devices and systems disclosed herein. The stent 20 includes an anchoring system such as one or more barbs or flaps formed therein, for example a first or distal flap 4a and a second or proximal flap 4b. The flaps 4a/4b may help to secure the stent 20 within the anatomy when the stent 20 is deployed. Other embodiments are contemplated where other anchoring systems are utilized. These other anchoring systems may be used instead of the flaps 4a/4b or in addition to the flaps 4a/4b.

The system 10 may be designed for use with a conventional guidewire 2 and may include a guide catheter 12, a push catheter 14, and a handle assembly 16. The guidewire 2 may extend into a lumen 22 of the guide catheter 12, through a distal guidewire port 24, and out a proximal guidewire port 26 formed in a sidewall of the push catheter 14 to a position where the guidewire 2 extends along the outer surface of the system 10. In at least some embodiments, the proximal guidewire port 26 may be disposed adjacent to a proximal end 32 of the push catheter 14 such that the system 10 is a "long wire" device. As the name implies, long wire devices utilize relatively long guidewires that extend along nearly the full length of the push catheter 14 and exit the proximal guidewire port 26, which is positioned near the handle assembly 16. For example, the proximal guidewire port 26 may be positioned about 0.1 to 10 cm or less, or about 1 to 5 centimeters or less from the proximal end of the push catheter 14. In other embodiments, the proximal guidewire port 26 may provide the system 10 with single-operator-exchange (SOE) capabilities such that shorter guidewire may be used. Other embodiments are also contemplated where the system 10 is an over-the-wire (OTW) system.

The guide catheter 12 may be slidably disposed within the lumen 28 of the push catheter 14 and may extend distally from the distal end of the push catheter 14. The stent 20 may be positioned on a distal portion of the guide catheter 12 (e.g., along an outer surface of the guide catheter 12), which may be located distal of the push catheter 14, and the stent 20 may abut the distal end 30 of the push catheter 14. The system 10 may also include a holding filament or suture (not shown) for releasably connecting the push catheter 14 to the stent 20. When the stent 20 has been properly placed within the anatomy, the stent 20 may be disconnected from the push catheter 14 such that the stent 20 remains in the anatomy or body lumen when the push catheter 14 is withdrawn.

The proximal end 32 of the push catheter 14 may be attached to the handle assembly 16. For example, the proximal end 32 may include a female luer lock connector 34 threadably coupled to a threaded male connector 36 of the handle assembly 16. It may be understood, however, that the push catheter 14 may be attached to the handle assembly 16 and extend distally therefrom by other means, such as adhesive bonding, welding, friction fit, interlocking fit, or other suitable means.

The guide catheter 12 may include a distal tubular portion 38 and a proximal elongate wire 40, such as a pull wire, coupled to the distal tubular portion 38. In some instances, the elongate wire 40 may be a wire, filament, thread, portion of a catheter wall, fabric, web, or similar elongate structure. The elongate wire 40 may be coupled to the distal tubular portion 38 at a rotatable connection that may allow rotatable movement between the tubular portion 38 and the elongate wire 40 of the guide catheter 12. The elongate wire 40 may extend through the lumen 28 of the push catheter 14 to the handle assembly 16. In some embodiments, the elongate wire 40 may extend through the handle assembly 16 to a location proximal of the handle assembly 16. The proximal end of elongate wire 40 may terminate at a knob 42 which may be grasped by an operator to manipulate the guide catheter 12.

As shown in Figure 2, the elongate wire 40 may share the lumen 28 of the push catheter 14 with the guidewire 2 along a portion of the length of the elongate wire 40. Thus, a portion of the elongate wire 40 may extend proximally from the tubular portion 38 along the side of the guidewire 2 through the lumen 28 of the push catheter 14 up to a location where the guidewire 2 exits the proximal guidewire port 26 of the push catheter 14.

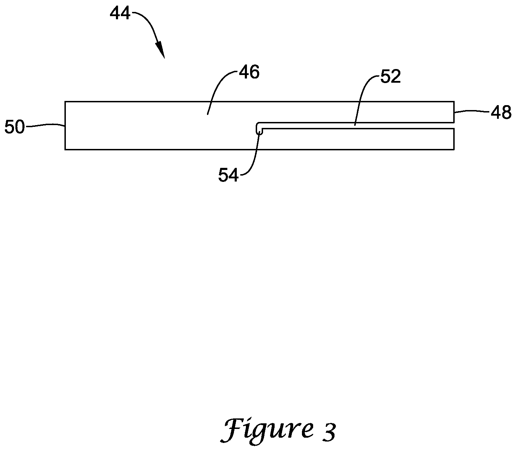

Figure 3 illustrates an example combination device 44, which is used in conjunction with or otherwise is part of the system 10. The combination device 44 may have several desirable uses. For example, the combination device 44 may be used to aid of the loading of the stent 20 into another medical device used with the system 10. This may include covering the anchoring mechanism of the stent 20 (e.g., flaps 4a/4b) with the combination device 44 during loading of the stent 20. For example, the combination device 44 may be disposed over the anchoring mechanism and compress the anchoring mechanism so that the stent 20 can be loaded into another medical device.

In addition, the combination device 44 may be used to secure the position of the guidewire 2 relative to the system 10. To do so, the position of the combination device 44 may be secured relative to the system 10. This may be done in a number of different ways. For example, the inner diameter of the combination device 44 may approximate the outer diameter of the system 10 (e.g., the outer diameter near or at the proximal end of the system 10). As such, the combination device 44 may be frictionally engaged with and held to the system 10 when positioned at the proximal end of the system 10. In other embodiments, a protrusion or hub may be formed on the system 10 such that the combination device 44 may be fitted over the protrusion and be held via friction and/or an interference fit. In still other embodiments, having the guidewire 2 within the combination device 44 may increase the friction between the combination device 44 and the system 10 or otherwise create an interference fit or bond. These are just examples. Numerous other securing relationships may be formed between the combination device and the system 10, which may allow the combination device to secure the position of the guidewire 2 relative to the system.

In general, the combination device 44 may be sized and shaped so as to fit over the stent 20 and/or the push catheter 14. In other words, the inner diameter of the combination device 44 may be about the same or slightly larger than the outer diameter of the stent 20 and/or the push catheter 14. In some embodiments, the inner diameter of the combination device 44 may be about 2.5 to about 10.2 mm (about 0.1 to about 0.4 inches), or about 2.667 to about 9.982 mm (about 0.105 to about 0.393 inches) (e.g., about 8-30 Fr), or about 2.667 to about 5.004 mm (about 0.105 to about 0.197 inches)
(e.g., about 8-15 Fr), or about 2.667 to about 3.327 mm (about 0.105 to about 0.131 inches) (e.g., about 8-10 Fr). These are just examples.

The combination device 44 includes a tubular body 46 having a proximal end 48 and a distal end 50. A locking slot 52 may be formed in the tubular body 46. In at least some embodiments, the locking slot 52 may extend from the proximal end 48 of the tubular body 46 toward the distal end 50. In at least some embodiments, the guidewire 2 may simply be pulled or wedged into the locking slot 52 so that the guidewire 2 is secured via an interference fit. In some of these and in other embodiments, a locking notch or aperture 54 may be formed at a distal portion of the locking slot 52. The locking notch 54 may be used to secure the position of the guidewire 2. For example, the locking notch 54 may be sized and/or shaped so that a clinician may pull the guidewire 2 into the locking notch 54 (e.g., via rotation of the combination device relative to the guidewire 2) in order to secure the position of the guidewire 2 relative to the system 10. Again, the guidewire 2 may be secured by an interference fit with the locking notch 54. The form and/or shape of the locking notch 54 may vary. For example, the locking notch may be round, oval, semicircular, semi-oval, polygonal, or have any suitable shape. In addition, the locking slot 52 and/or the locking notch 54 may be used to secure devices (e.g., catheters, etc.) other than or in addition to the guidewire 2. In some embodiments, multiple locking slots 52 and/or the locking notches 54 may be incorporated into the combination device 44 and each of the locking slots/notches 52/54 may be used to secure the same or different devices.

The combination device 44 provides a locking mechanism that may be conveniently located about (e.g., "on") the system 10 and that may be movable relative to (e.g., "along") the system 10. Because of this, a clinician may have access to, for example, a guidewire lock during an intervention. This convenient feature may also be desirable for other devices and/or interventions. For example, it may also be desirable to use the combination device 44 with medical device systems (e.g., catheters, endoscopic retrograde cholangiopancreatography devices, cutting devices including sphincterotomes, needle devices, balloon devices, brush devices, basket devices, snare devices, self-expanding stent delivery systems, and the like) other than just stent delivery systems (e.g., drainage stent delivery systems) where guidewire locking is desired. In other words, the combination device 44 may be used as a guidewire lock that can be disposed on essentially any medical device or device system.

In at least some embodiments, the combination device 44 may have a length that is relatively short (when compared to the overall length of the system 10). For example, the combination device 44 may have a length that is shorter than the length of the stent 20 or about the same as the length of the stent 20. Other embodiments, however, are contemplated where the length of the combination device 44 is longer. For example, the combination device may have a length that is longer than the length of the stent 20 and/or may span substantially the full length of the push catheter 14 (and/or the full length of the system 10). These are just examples.

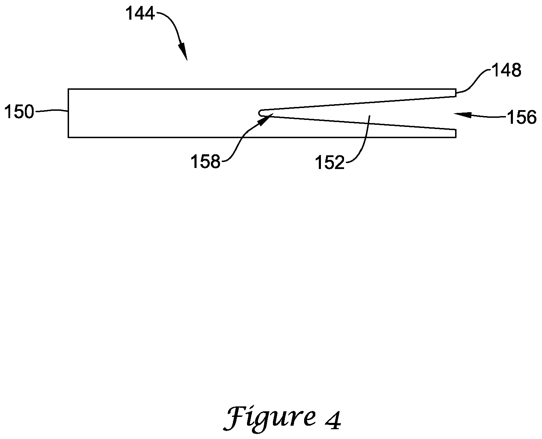

An alternative combination device 144 is illustrated in Figure 4. The combination device 144 has a proximal end 148, a distal end 150, and a locking slot 152. The locking slot 152 may taper such that a proximal portion 156 differs in width from a distal portion 158. For example, the proximal portion 156 may be wider than the distal portion 158. This may allow the combination device 144 to lock the guidewire 2 by, for example, wedging the guidewire 2 into the distal portion 158, where the guidewire 2 may be held by a friction fitting or interference fit.

Other embodiments are contemplated that are similar to what is shown, for example, in Figure 4. For example, in some embodiments the locking slot 152 may taper so as to define a slot or slit where the opposing edges of the slit touch or approximate each other. In some embodiments, the edges of the slit may overlap one another. The slit may be disposed along a portion of the length of the locking slot 152 or along substantially the full length of the locking slot 152. These are just examples as numerous configurations are contemplated.

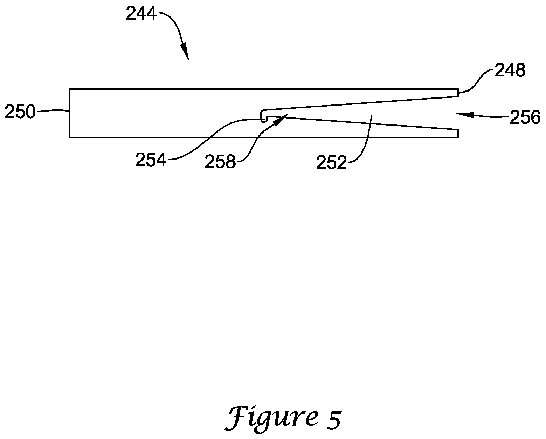

Another example combination device 244 is shown in Figure 5. The combination device 244 has a proximal end 248, a distal end 250, and a locking slot 252. The locking slot 252 may taper such that a proximal portion 256 differs in width from a distal portion 258. For example, the proximal portion 256 may be wider than the distal portion 258. This may allow the combination device 244 to lock the guidewire 2 by, for example, wedging the guidewire 2 into the distal portion 258, where the guidewire 2 may be held by a friction fitting or interference fit. In addition, the combination device 244 may also include a locking notch 254 similar to the locking notch 54. The locking notch 254 may be used to secure the position of the guidewire 2 in a manner similar to locking notch 54.

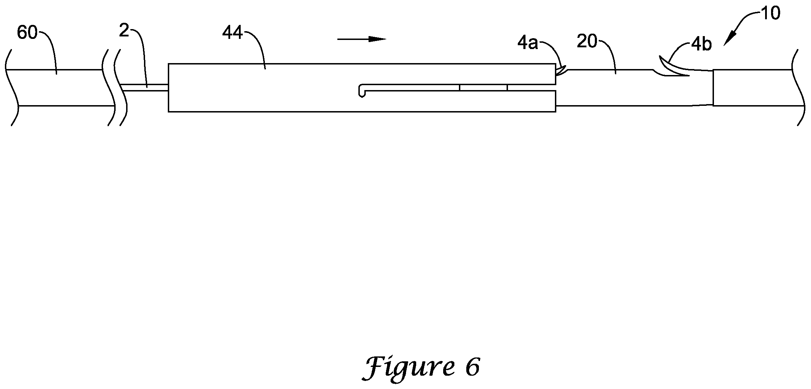

Figure 6 illustrates one of the uses contemplated for the combination device 44. It should be noted that while Figures 6-9 illustrate the use of the combination device 44, it can be appreciated that other combination devices disclosed herein, including the combination devices 144/244, or any other suitable combination device, may be used in a similar manner without departing from the spirit of the invention.

In Figure 6, it can be seen that the combination device 44 may be passed over the distal end of the system 10. Indeed, the combination device 44 can be urged proximally until it passes over the stent 20. This may include covering and/or compressing the anchoring mechanism (e.g., flaps 4a/4b) on the stent 20, which may make it easier for the stent 20 (and/or the system 10) to be advanced into another medical device 60, which may take the form of an endoscope. In Figure 6, the distal flap 4a can be seen being compressed. As the combination device 44 is urged further proximally, the combination device 44 can also compress the proximal flap 4b of the stent 20. Alternatively, the combination device 44 may be held substantially stationary and the system 10 may be advanced therethrough.

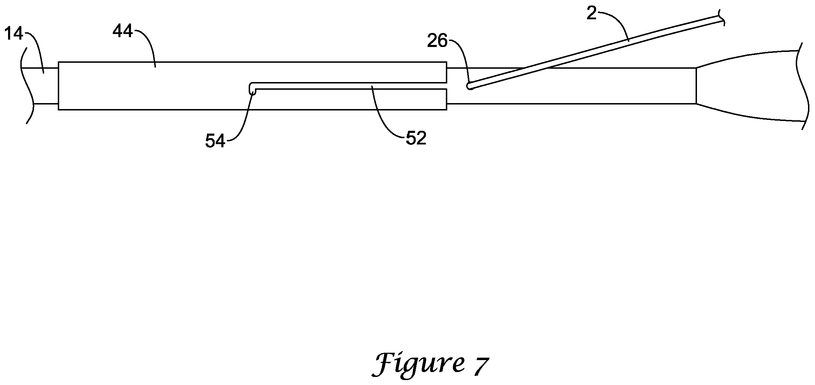

When the flaps 4a/4b of the stent 20 are suitably compressed such that the stent 20 (and/or the system 10) can be loaded into the endoscope 60, the combination device 44 may be further proximally advanced along the push catheter 14 to a position adjacent the proximal guidewire port 26 as shown in Figure 7. When positioned adjacent to the proximal guidewire port 26, the combination device 44 may find another one of its many uses. For example, the combination device 44 may be used to help secure the guidewire 2 relative to the system 10. This may be desirable for a number of reasons. For example, it may be relatively challenging to place the guidewire 2 within the anatomy of a patient. Therefore, maintaining the position of the guidewire 2 may help reduce the likelihood that additional guidewire placement steps, which may be technically challenging, would be necessary.

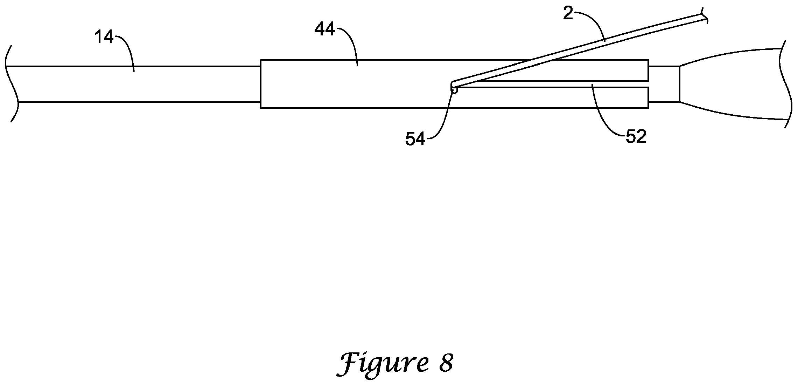

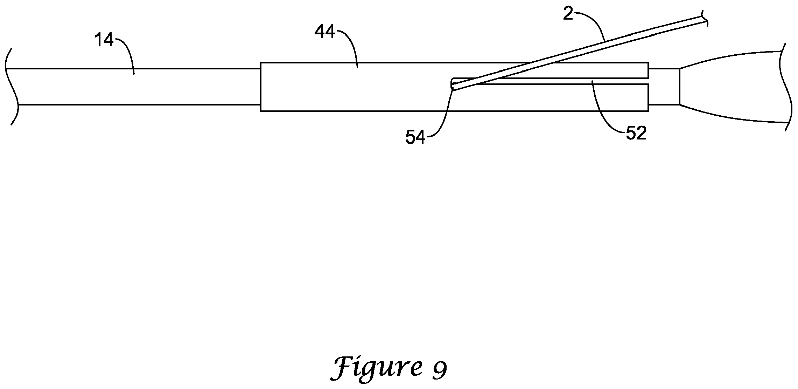

In order to use the combination device 44 to lock the guidewire 2, the combination device 44 can be positioned so that the guidewire 2 can be disposed within the locking slot 52. This may include rotating the combination device 44 so that the locking slot 52 is properly aligned with the guidewire 2. When properly aligned, the combination device 44 may be further proximally advanced so that the guidewire 2 enters the locking slot 52 as shown in Figure 8. Finally, the guidewire 2 can be secured by pulling the guidewire 2 into the locking notch 54 as shown in Figure 9.

Unlocking the guidewire 2 may involve the clinician simply removing the guidewire 2 from the locking notch 54 and/or the locking slot 52.

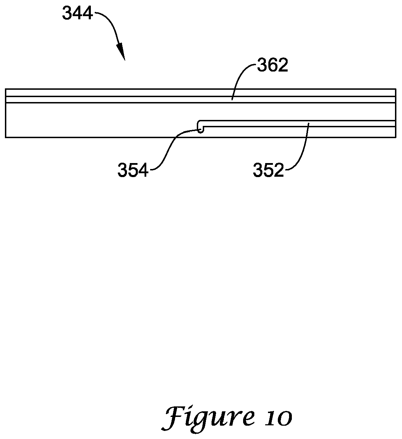

While Figures 6-9 illustrates that the combination device 44 may be used by disposing the combination device 44 essentially coaxially about the system 10, this is not intended to be limiting. For example, in at least some embodiments, the combination device 44 may be attachable to (and detachable with) the system 10. The precise structural configuration of the combination device 44 and/or the system 10 that permits attachment therebetween may vary. For example, Figure 10 illustrates an example combination device 344 that includes a loading slot 362 that spans the full length of the combination device 344 and allows the combination device 344 to be attached to the system (e.g., by laterally passing the combination device 344 over the system 10). The precise form of the loading slot 362 may vary. For example, the loading slot 362 may take the form of cut, slot, slit, spiral, or other shaped cut. These examples may include a cut where opposite sides of the wall of the combination device are disposed adjacent to one another or where the opposite sides of wall overlap one another. In some embodiments, the loading slot 362 may also be used to lock the guidewire 2 (e.g., the loading slot 362 may include a locking notch or other guidewire securing feature). In some of these and in other embodiments, the combination device 344 may include a locking slot 352 having a locking notch 354 or other guidewire securing feature formed therein.

In at least some embodiments, rather than using a "singular" combination device, a plurality of individual devices may be used to accomplish the various functions of the combination device. For example, a first device may be a tubular structure that is used, for example, to cover the anchoring mechanism (e.g., the flaps 4a/4b) during loading of the stent 20 into another device (e.g., an endoscope). This device may be tubular in form. A second device may be used as a device and/or guidewire securing device. The second device may also be tubular in form.

The materials that can be used for the various components of the system 10 and/or the various combination devices 44/144 disclosed herein may include those commonly associated with medical devices. For simplicity purposes, the following discussion makes reference to the push catheter and the combination device 44. However, this is not intended to limit the devices and methods described herein, as the discussion may be applied to any of the other structures and/or components of the delivery devices disclosed herein.

The combination device 44 and/or other components of the delivery system 10 may be made from a metal, metal alloy, polymer (some examples of which are disclosed below), a metal-polymer composite, ceramics, combinations thereof, and the like, or other suitable material. Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; titanium; combinations thereof; and the like; or any other suitable material.

The combination device 44 and/or other components of the delivery system 10 may include support and/or reinforcing structures incorporated therein such as, for example, a braid, a coil, a mesh, supporting fillers and/or amalgams, or the like. In addition, the combination device 44 and/or other components of the delivery system 10 may include cuts, slots, holes, openings, or the like formed therein, which may increase the flexibility.

In at least some embodiments, portions or all of the push catheter 14 and/or other components of the delivery system 10 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of the delivery system 10 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the delivery system 10 to achieve the same result.

In some embodiments, a degree of Magnetic Resonance Imaging (MRI) compatibility is imparted into the delivery system 10. For example, to enhance compatibility with MRI machines, it may be desirable to make the push catheter 14, or other portions of the delivery system 10, in a manner that would impart a degree of MRI compatibility. For example, the push catheter 14, or portions thereof, may be made of a material that does not substantially distort the image and create substantial artifacts (i.e., gaps in the image). Certain ferromagnetic materials, for example, may not be suitable because they may create artifacts in an MRI image. The push catheter 14, or portions thereof, may also be made from a material that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

The combination device 44 and/or other component the system 10 may be made from or otherwise include a polymer or polymeric material. Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane (for example, Polyurethane 85A), polypropylene (PP), polyvinylchloride (PVC), polyether-ester (for example, ARNITEL9 available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, polyolefin, polystyrene, epoxy, polyvinylidene chloride (PVdC), poly(styrene*-b-*isobutylene*-b-*styrene) (for example, SIBS and/or SIBS 50A), polycarbonates, ionomers, biocompatible polymers, other suitable materials, or mixtures, combinations, copolymers thereof, polymer/metal composites, and the like. In some embodiments the polymer can be blended with a liquid crystal polymer (LCP). For example, the mixture can contain up to about 6 percent LCP.

In some embodiments, the exterior surface of the delivery system 10 may be sandblasted, beadblasted, sodium bicarbonate-blasted, electropolished, etc. In these as well as in some other embodiments, a coating, for example a lubricious, a hydrophilic, a protective, or other type of coating may be applied over portions or all of the delivery system 10. Hydrophobic coatings such as fluoropolymers provide a dry lubricity which improves guidewire handling and device exchanges. Lubricious coatings improve steerability and improve lesion crossing capability. Suitable lubricious polymers are well known in the art and may include silicone and the like, hydrophilic polymers such as high-density polyethylene (HDPE), polytetrafluoroethylene (PTFE), polyarylene oxides, polyvinylpyrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility. Some other examples of such coatings and materials and methods used to create such coatings can be found in U.S. Patent Nos. 6,139,510 and 5,772,609.

The coating and/or sheath may be formed, for example, by coating, extrusion, co-extrusion, interrupted layer co-extrusion (ILC), gradient extrusion, or fusing several segments end-to-end. The layer may have a uniform stiffness or a gradual reduction in stiffness from the proximal end to the distal end thereof. The gradual reduction in stiffness may be continuous as by ILC or may be stepped as by fusing together separate extruded tubular segments. The outer layer may be impregnated with a radiopaque filler material to facilitate radiographic visualization. Those skilled in the art will recognize that these materials can vary widely without deviating from the scope of the present invention.

The arrangement of the various structures of the system 10 may vary. In some embodiments, the system 10 may include any of the structures or utilize any of the arrangements of structures that are disclosed in U.S. Patent Nos. 5,152,749; 5,334,185; 5,921,952; 6,248,100; 6,264,624; and 6,562,024.

It should be understood that this disclosure is, in many respects, only illustrative. Changes may be made in details, particularly in matters of shape, size, and arrangement of steps without exceeding the scope of the invention as defined by the claims. The invention's scope is, of course, defined in the language in which the appended claims are expressed.

## Claims

1. An assembly comprising:
a guidewire (2);
a drainage stent (20) having an anchoring mechanism (4a, 4b);
a catheter system (12, 14, 16); and
a combination device (44; 144; 244; 344) including an anchoring mechanism cover, the combination device comprising a tubular body (46), the tubular body defining an inner diameter and having a locking portion (52, 54; 152; 252, 254; 352, 354);
wherein the inner diameter of the tubular body (46) is sized to fit about an outer diameter of the drainage stent (20) and is sized to cover the anchoring mechanism (4a,4b); and
wherein the locking portion (52, 54; 152; 252, 254; 352, 354) of the tubular body (46) is configured to secure the position of the guidewire (2) relative to the catheter system (12, 14, 16).

2. The assembly of claim 1, wherein the anchoring mechanism (4a, 4b) includes one or more flaps formed in the stent (20).

3. The assembly of any one of claims 1-2, wherein the locking portion (52, 54; 152; 252, 254; 352, 354) includes a locking slot (52; 152; 252; 352) formed in the tubular body.

4. The assembly of claim 3, wherein a locking notch (54; 254; 354) is formed in the tubular body (46) and positioned adjacent to the locking slot (52; 252; 352).

5. The assembly of claim 3, wherein the locking slot (152; 252) includes a first portion (156; 256) and a second portion (158; 258), the first portion (156; 256) being wider than the second portion (158; 258).

6. The assembly of any one of claims 1-5, wherein the locking portion (52, 54; 152; 252, 254; 352, 354) is configured to be rotated relative to the guidewire (2) to secure the position of the guidewire (2).

7. The assembly of any one of claims 1-6, wherein the locking portion (52, 54; 152; 252, 254; 352, 354) creates an interference fit with the guidewire (2).

8. The assembly of any one of claims 1-7, wherein the stent (20) is disposed about a guide catheter (12) of the catheter system (12, 14, 16).

9. The assembly of claim 8, wherein a push catheter (14) of the catheter system (12, 14, 16) is disposed about the guide catheter (12) and positioned adjacent to the stent (20).

10. The assembly of claim 9, wherein the guide catheter (12), the push catheter (14), or both are configured to extend through an endoscope (60).

11. The assembly of any one of claims 1-10, wherein the combination device (344) has a loading slot (362) extending along the length thereof.

## Patentansprüche

1. Baugruppe, aufweisend:
einen Führungsdraht (2);
einen Drainage-Stent (20), der einen Verankerungsmechanismus (4a, 4b) aufweist;
ein Kathetersystem (12, 14, 16); und
ein Kombinationsgerät (44; 144; 244; 344), das eine Verankerungsmechanismus-Abdeckung aufweist, wobei das Kombinationsgerät einen Rohrkörper (46) aufweist, wobei der Rohrkörper einen Innendurchmesser definiert und einen Verriegelungsabschnitt (52, 54; 152; 252, 254; 352, 354) aufweist;
wobei der Innendurchmesser des Rohrkörpers (46) so bemessen ist, dass er um einen Außendurchmesser des Drainage-Stents (20) passt, und für eine Bedeckung des Verankerungsmechanismus (4a, 4b) bemessen ist; und
wobei der Verriegelungsabschnitt (52, 54; 152; 252, 254; 352, 354) des Rohrkörpers (46) zum Sichern der Position des Führungsdrahts (2) in Bezug auf das Kathetersystem (12, 14, 16) ausgebildet ist.

2. Baugruppe nach Anspruch 1, wobei der Verankerungsmechanismus (4a, 4b) eine oder mehrere im Stent (20) gebildete Klappen aufweist.

3. Baugruppe nach einem der Ansprüche 1-2, wobei der Verriegelungsabschnitt (52, 54; 152; 252, 254; 352, 354) einen im Rohrkörper gebildeten Verriegelungsschlitz (52; 152; 252; 352) aufweist.

4. Baugruppe nach Anspruch 3, wobei im Rohrkörper (46) eine Verriegelungskerbe (54; 254; 354) gebildet ist, die benachbart zum Verriegelungsschlitz (52; 252; 352) angeordnet ist.

5. Baugruppe nach Anspruch 3, wobei der Verriegelungsschlitz (152; 252) einen ersten Abschnitt (156; 256) und einen zweiten Abschnitt (158; 258) aufweist, wobei der erste Abschnitt (156; 256) breiter als der zweite Abschnitt (158; 258) ist.

6. Baugruppe nach einem der Ansprüche 1-5, wobei der Verriegelungsabschnitt (52, 54; 152; 252, 254; 352, 354) dazu ausgebildet ist, in Bezug auf den Führungsdraht (2) gedreht zu werden, um die Position des Führungsdrahts (2) zu sichern.

7. Baugruppe nach einem der Ansprüche 1-6, wobei der Verriegelungsabschnitt (52, 54; 152; 252, 254; 352, 354) eine Presspassung mit dem Führungsdraht (2) erzeugt.

8. Baugruppe nach einem der Ansprüche 1-7, wobei der Stent (20) um einen Führungskatheter (12) des Kathetersystems (12, 14, 16) angeordnet ist.

9. Baugruppe nach Anspruch 8, wobei ein Vorschubkatheter (14) des Kathetersystems (12, 14, 16) um den Führungskatheter (12) angeordnet und benachbart zum Stent (20) positioniert ist.

10. Baugruppe nach Anspruch 9, wobei der Führungskatheter (12), der Vorschubkatheter (14) oder beide dazu ausgebildet sind, sich durch ein Endoskop (60) zu erstrecken.

11. Baugruppe nach einem der Ansprüche 1-10, wobei das Kombinationsgerät (344) einen Ladeschlitz (362) aufweist, der sich über dessen Länge erstreckt.

## Revendications

1. Ensemble comprenant :
un fil-guide (2) ;
un stent de drainage (20) ayant un mécanisme d'ancrage (4a, 4b) ;
un système de cathéter (12, 14, 16) ; et
un dispositif de combinaison (44 ; 144 ; 244 ; 344) comprenant un couvercle de mécanisme d'ancrage, le dispositif de combinaison comprenant un corps tubulaire (46), le corps tubulaire définissant un diamètre interne et ayant une partie de verrouillage (52, 54 ; 152 ; 252, 254 ; 352, 354) ;
dans lequel le diamètre interne du corps tubulaire (46) est dimensionné pour s'adapter autour d'un diamètre externe du stent de drainage (20) et est dimensionné pour recouvrir le mécanisme d'ancrage (4a, 4b) ; et
dans lequel la partie de blocage (52, 54 ; 152 ; 252, 254 ; 352, 354) du corps tubulaire (46) est configurée pour garantir la position du fil-guide (2) par rapport au système de cathéter (12, 14, 16).

2. Ensemble selon la revendication 1, dans lequel le mécanisme d'ancrage (4a, 4b) comprend un ou plusieurs volets formés dans le stent (20).

3. Ensemble selon l'une quelconque des revendications 1 à 2, dans lequel la partie de blocage (52, 54 ; 152 ; 252, 254 ; 352, 354) comprend une fente de blocage (52 ; 152 ; 252 ; 352) formée dans le corps tubulaire.

4. Ensemble selon la revendication 3, dans lequel une encoche de blocage (54 ; 254 ; 354) est formée dans le corps tubulaire (46) et positionnée de manière adjacente à la fente de blocage (52 ; 252 ; 352).

5. Ensemble selon la revendication 3, dans lequel la fente de blocage (152 ; 252) comprend une première partie (156 ; 256) et une seconde partie (158 ; 258), la première partie (156 ; 256) étant plus large que la seconde partie (158 ; 258).

6. Ensemble selon l'une quelconque des revendications 1 à 5, dans lequel la partie de blocage (52, 54 ; 152 ; 252, 254 ; 352 ; 354) est configurée pour être entraînée en rotation par rapport au fil-guide (2) afin de garantir la position du fil-guide (2).

7. Ensemble selon l'une quelconque des revendications 1 à 6, dans lequel la partie de blocage (52, 54 ; 152 ; 252, 254 ; 352, 354) crée un ajustement avec serrage avec le fil-guide (2).

8. Ensemble selon l'une quelconque des revendications 1 à 7, dans lequel le stent (20) est disposé autour d'un cathéter de guidage (12) du système de cathéter (12, 14, 16).

9. Ensemble selon la revendication 8, dans lequel un cathéter de poussée (14) du système de cathéter (12, 14, 16) est disposé autour du cathéter de guidage (12) et positionné de manière adjacente au stent (20).

10. Ensemble selon la revendication 9, dans lequel le cathéter de guidage (12), le cathéter de poussée (14) ou les deux sont configurés pour s'étendre à travers un endoscope (60).

11. Ensemble selon l'une quelconque des revendications 1 à 10, dans lequel le dispositif de combinaison (344) a une fente de charge (362) s'étendant le long de sa longueur.
